# EUROPÄISCHE PATENTSCHRIFT

(11) **EP 3 937 782 B1**
(45) Veröffentlichungstag und Bekanntmachung des Hinweises auf die Patenterteilung: **07.01.2026**
(21) Anmeldenummer: 19729639.5
(22) Anmeldetag: 24.05.2019
(51) Int. Cl.: A61B 6/00, B25J 13/02, G01L 5/22

(54) **MOBILES MEDIZINISCHES GERÄT UND VERFAHREN**
MOBILE MEDICAL DEVICE AND METHOD
APPAREIL MÉDICAL MOBILE ET PROCÉDÉ

(43) Veröffentlichungstag der Anmeldung: 19.01.2022
(73) Patentinhaber: Siemens Healthineers AG, 91301 Forchheim (DE)
(72) Erfinder: DIRAUF, Franz, 96231 Bad Staffelstein (DE); GEMMEL, Alexander, 91056 Erlangen (DE); REINHARD, Malte, 90425 Nürnberg (DE); ZEIDLER, Josef, 95615 Marktredwitz (DE)
(74) Vertreter: Siemens Healthineers Patent Attorneys
(86) Internationale Anmeldenummer: PCT/EP2019/063459
(87) Internationale Veröffentlichungsnummer: WO 2020/239187

(56) Entgegenhaltungen:
- EP-A1- 3 257 463
- JP-A- 2011 167 296
- US-A1- 2017 215 826
- US-B1- 6 276 485

## Beschreibung

Die Erfindung betrifft ein mobiles medizinisches Gerät mit einem in mindestens zwei Bewegungsmodi motorisch unterstützt verfahrbaren Gerätewagen gemäß dem Patentanspruch 1sowie ein Verfahren zur Bedienunterstützung eines derartigen mobilen medizinischen Geräts gemäß dem Patentanspruch 11.

Mobile Röntgengeräte, welche auf Gerätewagen angeordnet und mit Rädern versehen sind und sich so über den Krankenhausboden bewegen lassen, weisen eine besonders große Flexibilität bezüglich ihrer Einsetzbarkeit in unterschiedlichen Bereichen oder Räumen auf. Hierbei muss eine in der Praxis gut handhabbare manuelle Verfahrbarkeit gegeben sein, insbesondere muss das Gerät von einer Bedienperson lenk- bzw. steuerbar und bremsbar sein. Dabei ist zudem darauf zu achten, dass das Gerät beim Verfahren möglichst leicht kontrollierbar ist und die für die Kontrolle notwendigen Einrichtungen einen kompakten Aufbau des Gerätes erlauben, um den Raumbedarf des Gerätes möglichst gering zu halten.

Neuere mobile, handgesteuerte Röntgengeräte weisen eine motorisch unterstützte Verfahrbarkeit auf, um der Bedienperson die Bedienung bzw. den Transport des Gerätes zu erleichtern.

Außerdem haben moderne handgesteuerte, motorisierte Röntgengeräte je nach Grad und Art und Anordnung ihrer Räder mehrere Freiheitsgrade. In Abhängigkeit des Nutzungskontexts ist es wünschenswert, einzelne Freiheitsgrade zu bevorzugen oder zu sperren. Beispielsweise soll ein mobiles Rötgengerät beim Fahren über den Gang von einem Operationsraum zu einem anderen Operationsraum hauptsächlich in Vorwärtsrichtung und in Kurven schnell bewegt werden, während es im Operationsraum am OP-Tisch eher langsam und präzise vorwärts und seitwärts bewegt werden soll. Mobile Geräte bieten daher häufig die Möglichkeit, bestimmte Bewegungsmodi (z.B. Bewegung nur in Vorwärtsrichtung) mittels Schalter oder Bedienknopf auszuwählen, um dem System mitzuteilen, in welchem Bewegungsmodus er es bewegen mochte. Auch Bewegungsprofile (Beschleunigung, Geschwindigkeiten) können so eingestellt werden.

Aus der Veröffentlichungsschrift US 2017/215826 A1 ist ein mobiles medizinisches Gerät mit einem in mindestens zwei Bewegungsmodi motorisch unterstützt verfahrbaren Gerätewagen mit zwei Griffelementen, die jeweils zwei Sensoren aufweisen, bekannt. Die Veröffentlichungsschrift JP 2011167296 A offenbart ebenfalls ein mobiles medizinisches Gerät mit einem in mindestens zwei Bewegungsmodi motorisch unterstützt verfahrbaren Gerätewagen mit einem Griffelement, welches als Schalter fungiert.

Es ist Aufgabe der vorliegenden Erfindung, ein mobiles medizinisches Gerät mit einem in mindestens zwei Bewegungsmodi motorisch unterstützt verfahrbaren Gerätewagen bereitzustellen, welches eine verbesserte Bedienbarkeit des Gerätes ermöglicht; des Weiteren ist es Aufgabe der Erfindung, ein Verfahren zur Bedienungsunterstützung des Gerätes bereitzustellen.

Die Aufgabe wird erfindungsgemäß gelöst durch ein mobiles medizinisches Gerät mit einem in mindestens zwei Bewegungsmodi motorisch unterstützt verfahrbaren Gerätewagen gemäß dem Patentanspruch 1 und von einem Verfahren zur Bedienunterstützung eines derartigen mobilen medizinischen Geräts gemäß dem Patentanspruch 11. Vorteilhafte Ausgestaltungen der Erfindung sind jeweils Gegenstand der zugehörigen Unteransprüche.

Das erfindungsgemäße mobile medizinischen Gerät, insbesondere mobile C-Bogen-Röntgengerät, mit einem in mindestens zwei Bewegungsmodi motorisch unterstützt verfahrbaren Gerätewagen, aufweisend mindestens ein Griffelement, wobei das mindestens eine Griffelement für ein Greifen mittels mindestens einer menschlichen Hand einer Bedienperson zum Verfahren des Gerätes vorgesehen ist, weist außerdem mindestens einen Sensor, welcher an dem Griffelement oder in dessen unmittelbarer Nähe angeordnet und dafür ausgebildet ist, die Art und/oder den Ort des Greifens mittels der mindestens einen menschlichen Hand charakterisierende Messdaten zu erfassen, eine Auswerteeinheit, welche dazu ausgebildet ist, die Messdaten hinsichtlich der Art und/oder des Ortes des Greifens auszuwerten und einem Bewegungsmodus des Gerätewagens zuzuordnen, und eine Steuerungseinheit zur automatischen Einstellung des ausgewerteten Bewegungsmodus des Gerätewagens auf.

Das mobile medizinische Gerät bzw. der Gerätewagen besitzt zur Erleichterung der Verfahrbarkeit eine bekannte motorische Unterstützung, d.h. Servounterstützung, welche dafür sorgt, dass der Kraftaufwand zum Bewegen des Gerätes für die Bedienperson deutlich reduziert ist. Unter einem Bewegungsmodus ist dabei eine bestimmte Voreinstellung zu verstehen, bei der ein oder mehrere Charakteristiken der Bewegbarkeit des Geräts (z.B. langsam fahren, schnell fahren, Translationen, Kurven, Seitwärtsbewegungen ... ) besonders unterstützt und/oder andere weniger oder gar nicht unterstützt bzw. blockiert werden. So kann zum Beispiel ein Bewegungsmodus dafür ausgelegt sein, ein schnelles Verfahren des Gerätewagens in Geradeausrichtung und/oder in Kurven zu unterstützen und eine Seitwärtsbewegung zu unterbinden, dieser Bewegungsmodus kann für einen Transport des Gerätes von einem Raum in einen anderen Raum vorgesehen sein. Ein anderer Bewegungsmodus kann dafür ausgelegt sein, aus dem Stand langsam kleine Entfernungen (z.B. im Zentimeterbereich) in alle möglichen Richtungen zurückzulegen, dieser Bewegungsmodus kann für Positionierungen des Geräts im Operationsraum vorgesehen sein. Ein weiterer Bewegungsmodus kann dafür ausgelegt sein, langsam mittlere Entfernungen (z.B. im Meterbereich) und Kurven zurückzulegen, dieser Bewegungsmodus kann für eine Bewegung des Geräts im Operationsraum in eine Parkposition vorgesehen sein.

Die Erfindung umfasst außerdem ein Verfahren zur automatischen Bedienunterstützung eines mobilen medizinischen Gerätes mit einem in mindestens zwei Bewegungsmodi motorisch unterstützt verfahrbaren Gerätewagen und zumindest einem Griffelement für ein Greifen mittels mindestens einer menschlichen Hand einer Bedienperson zum Verfahren des Gerätewagens mit den folgenden Schritten: Erfassen von die Art und/oder den Ort des Greifens mittels der mindestens einen menschlichen Hand charakterisierenden Messdaten, Auswerten der Messdaten hinsichtlich der Art und/oder des Ortes des Greifens und Zuordnen zu einem Bewegungsmodus des Gerätewagens, und Automatische Einstellung des ermittelten Bewegungsmodus des Gerätewagens.

Das erfindungsgemäße mobile medizinische Gerät und das erfindungsgemäße Verfahren weisen viele Vorteile auf. Durch die automatische Erkennung der Greifcharakteristik der Bedienperson und automatischen Umsetzung in einen Bewegungsmodus wird die Bedienung des mobilen Röntgengeräts vereinfacht und gestaltet sich für die Bedienperson besonders intuitiv. Es ergeben sich ein entscheidender Zeitgewinn und eine Steigerung der Effizienz bei medizinischen Untersuchungen. Die Bedienperson kann problemlos eine Blindbedienung ausführen, kann ihre Aufmerksamkeit auf andere wichtigere Aufgaben richten und muss sich nicht um eine manuelle Anwahl von Bewegungsprofilen kümmern, da dies von dem mobilen Gerät bereits automatisch durchgeführt wird. Die automatische Erkennung von Greifcharakteristiken und deren Zuordnung zu bestimmten Bewegungsmodi ist besonders zuverlässig und fehlersicher und erleichtert der Bedienperson die Bedienung des medizinischen Geräts in hohem Maße.

Nach einer Ausgestaltung der Erfindung ist oder sind der Sensor oder die Sensoren dazu ausgebildet, die Anzahl der greifenden Hände und/oder die Größe der greifenden Hand oder Hände und/oder die Position der greifenden Hand oder Hände und/oder die Auflagefläche der greifenden Hand oder Hände und/oder die Grifffestigkeit des Greifens und/oder ein Greifprofil einer Bedienperson charakterisierende Messdaten zu erfassen. Derartige Greifcharakteristiken sind besonders geeignet, Rückschlüsse auf eine von der Bedienperson geplante oder beabsichtigte Bewegung des Gerätes zu geben, so dass durch Erkennen einer oder mehrerer dieser Charakteristiken eine automatische Auswahl eines Bewegungsmodus besonders einfach und fehlersicher möglich ist.

Nach einer weiteren Ausgestaltung der Erfindung wird der zumindest eine Sensor von zumindest einem kapazitiven Sensor gebildet. Kapazitive Sensoren sind besonders zuverlässig und effektiv im Erkennen von Fingern oder Händen von Personen. Diese Sensoren können auch besonders einfach und kostengünstig in das Griffelement integriert werden. Insbesondere kann auch eine Vielzahl von Sensoren am oder in unmittelbarer Nähe des Griffelements angeordnet sein. Es kann auch das komplette Griffelement aus kapazitiven Sensoren aufgebaut sein, um eine besonders zuverlässige Erkennung zu gewährleisten. Zusätzlich können noch weitere Sensoren vorgesehen sein, zum Beispiel resistive Sensoren, Näherungssensoren, Lichtsensoren, Drucksensoren oder Kameras.

Erfindungsgemäß sind die mindestens zwei Bewegungsmodi für mindestens zwei Bedienpersonen personalisiert einstellbar, wobei der mindestens eine Sensor dazu ausgebildet ist, ein Greifprofil einer Bedienperson charakterisierende Messdaten zu erfassen, die Auswerteeinheit dazu ausgebildet ist, die Messdaten hinsichtlich der Bedienperson auszuwerten und einem personalisierten Bewegungsmodus zuzuordnen und die Steuerungseinheit zur automatischen Einstellung des ausgewerteten personalisierten Bewegungsmodus ausgebildet ist. Um dies zu erreichen, kann zum Beispiel über das Erfassen der Greifcharakteristik auch die Bedienperson erfasst werden, zum Beispiel über die Größe der Hände, und entsprechend der personalisierte Bewegungsmodus eingestellt werden. Alternativ kann auch über eine zusätzliche Kamera und eine Bilderkennungssoftware die Bedienperson erkannt werden und dann der Bewegungsmodus personalisiert eingestellt werden.

Nach einer weiteren Ausgestaltung der Erfindung ist ein erster Bewegungsmodus derart ausgebildet ist, dass bei Einstellen des ersten Bewegungsmodus ein schnelles Verfahren des Gerätewagens über Entfernungen > 2 m unterstützt wird. Nach einer weiteren Ausgestaltung der Erfindung ist ein zweiter Bewegungsmodus derart ausgebildet, dass bei Einstellen des zweiten Bewegungsmodus ein langsames Verfahren über Entfernungen unter 15 cm unterstützt wird. Dies sind im Allgemeinen die grundsätzlich wichtigsten Bewegungsmodi. Diese können bei Bedarf jedoch auch deutlich spezieller ausgebildet sein, zum Beispiel durch Blocken oder Unterstützen bestimmter anderer Bewegungsarten. Es können auch weitere Bewegungsmodi vorhanden sein, bei denen ein oder mehrere Charakteristiken der Bewegbarkeit des Geräts (z.B. langsam fahren, schnell fahren, Translationen, Kurven, Seitwärtsbewegungen ... ) besonders unterstützt und/oder andere weniger oder gar nicht unterstützt werden. Es kann auch eine Vielzahl verschiedener Bewegungsmodi vorhanden sein.

Das mobile medizinische Gerät wird nach einer Ausgestaltung der Erfindung von einem mobilen C-Bogen-Röntgengerät mit einem in mehreren Freiheitsgraden verstellbaren, rotierbaren C-Bogen gebildet.

Nach einer weiteren Ausgestaltung der Erfindung ist ein erstes Griffelement zum Anschieben und/oder Ziehen des in mindestens zwei Bewegungsmodi motorisch unterstützt verfahrbaren Gerätewagens ausgebildet und ein zweites Griffelement für ein Greifen mittels mindestens einer menschlichen Hand einer Bedienperson zum motorisch unterstützten Verstellen des C-Bogens ausgebildet. In diesem Zusammenhang kann vorgesehen sein, dass das zweite Griffelement mindestens einen Sensor aufweist, welcher an dem Griffelement oder in dessen unmittelbarer Nähe angeordnet und dafür ausgebildet ist, die Art und/oder den Ort des Greifens mittels der mindestens einen menschlichen Hand charakterisierende Messdaten zu erfassen, und wobei die Auswerteeinheit dazu ausgebildet ist, die Messdaten hinsichtlich der Art und/oder des Ortes des Greifens auszuwerten und einem Verstellmodus des C-Bogens zuzuordnen, und die Steuerungseinheit zur automatischen Einstellung des ausgewerteten Verstellmodus ausgebildet ist. Auch die Verstellbarkeit des C-Bogens besitzt in diesem Fall eine Servounterstützung. Unter einem Verstellmodus ist hierbei analog zu dem Bewegungsmodus eine bestimmte Voreinstellung zu verstehen, bei der ein oder mehrere Charakteristiken der Verstellbarkeit des C-Bogens (z.B. langsam verstellen, schnell verstellen, Rotationen, Translationen ... ) besonders unterstützt und/oder andere weniger oder gar nicht unterstützt bzw. blockiert werden. Durch das zweite Griffelement und die entsprechende Ausgestaltung des medizinischen Geräts wird die Bedienung des mobilen Geräts ebenfalls vereinfacht und gestaltet sich für die Bedienperson besonders intuitiv.

Nach einer weiteren Ausgestaltung der Erfindung weist das mobile medizinische Gerät eine Anzeigeeinheit zur Anzeige von Informationen bezüglich des aktivierten Bewegungsmodus auf. Diese Anzeige kann z.B. ebenfalls am oder im Griffelement integriert sein oder zumindest in unmittelbarer Nähe dazu angeordnet sein. Es kann sich hierbei zum Beispiel um eine LED-Anzeige handeln. Eine derartige Anzeigeeinheit weist den Vorteil auf, dass eine Bedienperson sofort auf einen Blick erkennen kann, welcher Bewegungsmodus automatisch ausgewählt wurde.

Insbesondere ist die Erfindung anwendbar für mobile Geräte mit hohem spezifischem Gewicht, um eine einfache Manövrierbarkeit zu gewährleisten.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: eine Ansicht eines erfindungsgemäßen mobilen C-Bogen-Röntgengeräts mit einem Griffelement;
- FIG 2: eine Ansicht eines Griffelements; und
- FIG 3: eine Abfolge eines erfindungsgemäßen Verfahrens zur automatischen Bedienunterstützung eines mobilen C-Bogen-Röntgengeräts.

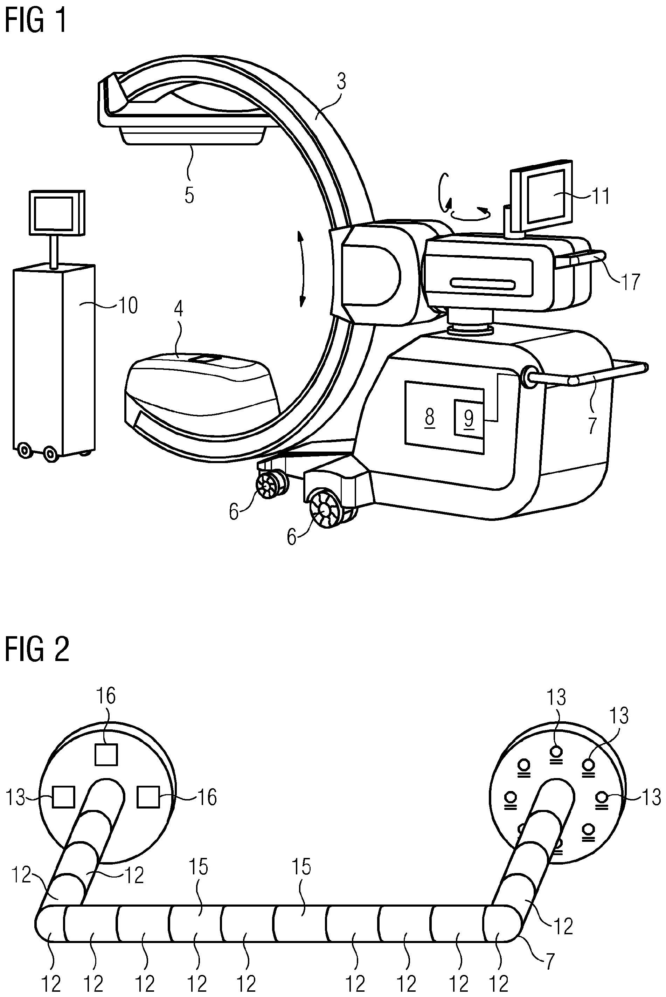

In der FIG 1 ist ein mobiles C-Bogen-Röntgengerät 1 gezeigt, welches einen auf einem Gerätewagen 2 angeordneten C-Bogen 3 aufweist. Der C-Bogen 3 ist mittels einer oder mehrerer mechanischer Verbindungen derart an dem Gerätewagen 2 angeordnet, dass der C-Bogen 3 in mehreren Freiheitsgraden verstellbar ist. So kann er zum Beispiel rotiert (siehe Pfeile), gekippt und translatiert werden. Am C-Bogen 3 ist an einem Ende eine Röntgenquelle 4 und an dem anderen Ende ein Röntgendetektor 5 angeordnet, mittels welchen Röntgenaufnahmen eines Untersuchungsobjekts in einer Vielzahl von möglichen Aufnahmepositionen (Angulationen) aufgenommen werden können. Das mobile C-Bogen-Röntgengerät 1 wird von einer Systemsteuerung 8 angesteuert. Der Gerätewagen 2 weist mehrere, zum Beispiel zwei, drei oder vier, multi- oder omni-direktionale Räder 6 auf, mittels denen er vorwärts, seitwärts und/oder in Kurven bewegt und verfahren werden kann. Die Räder können jeweils eigene Antriebe aufweisen. Im Allgemeinen wird das mobile C-Bogen-Röntgengerät von einer Bedienperson verschoben bzw. verfahren. Hierfür weist der Gerätewagen ein Griffelement 7 auf, welches eine Bedienperson mit einer oder beiden Händen umfassen kann, um den Gerätewagen zu schieben oder zu ziehen. Es können auch zwei oder mehr Griffelemente an dem Gerätewagen angeordnet sein. Die Verfahrbarkeit ist zusätzlich kraftunterstützt, das Gerät weist also einen (bekannten, nicht gezeigten) Servoantrieb auf, der die Bedienperson beim Verfahren motorisch unterstützt. Auf diese Weise kann das mobile C-Bogen-Röntgengerät 1 einfach in alle Richtungen verfahren werden.

Im Rahmen der motorisch unterstützten Verfahrbarkeit weist das mobile C-Bogen-Röntgengerät 1 mindestens zwei verschiedene Bewegungsmodi auf. Unter einem Bewegungsmodus ist dabei eine bestimmte Voreinstellung zu verstehen, bei der ein oder mehrere Charakteristiken der Bewegbarkeit des Gerätewagens (z.B. langsam fahren, schnell fahren, Translationen, Kurven, Seitwärtsbewegungen ... ) besonders unterstützt und/oder andere weniger oder gar nicht unterstützt bzw. blockiert werden. So kann zum Beispiel ein erster Bewegungsmodus dafür ausgelegt sein, ein schnelles Verfahren des Gerätewagens in Geradeausrichtung und/oder in Kurven zu unterstützen und eine Seitwärtsbewegung zu unterbinden, dieser Bewegungsmodus kann z.B. für einen Transport des Gerätewagens über Strecken > 4 m, z.B. von einem Raum in einen anderen Raum, vorgesehen sein. Ein anderer (z.B. zweiter) Bewegungsmodus kann dafür ausgelegt sein, aus dem Stand langsam kleine Entfernungen (z.B. unter 50 cm) in alle möglichen Richtungen zurückzulegen, dieser Bewegungsmodus kann für (Fein-)Positionierungen des Geräts im Operationsraum vorgesehen sein. Ein weiterer (z.B. dritter) Bewegungsmodus kann dafür ausgelegt sein, langsam mittlere Entfernungen (z.B. zwischen 50 cm und 4 m) und Kurven zurückzulegen, dieser Bewegungsmodus kann für eine Bewegung des Geräts im Operationsraum in eine Parkposition vorgesehen sein. Es können auch noch weitere oder alternative Bewegungsmodi mit z.B. nur einem Charakteristikum (schnell, langsam, usw.) vorhanden sein.

Für eine besonders intuitive Bedienung in Bezug auf die Verfahrbarkeit weist das C-Bogen-Röntgengerät mehrere Komponenten auf, welche eine automatische Einstellung des aktuell benötigten Bedienmodus gewährleistet. So sind eine Vielzahl von kapazitiven Sensoren 12 in das Griffelement 7 integriert - wie z.B. auch in der FIG 2 vergrößert gezeigt. Die kapazitiven Sensoren 12 sind ausgebildet, Messdaten bezüglich des Greifens der Bedienperson zu erfassen, also z.B. wo genau und an wie vielen Positionen des Griffelements 7 gegriffen wird. Die Messdaten werden dann an eine Auswerteeinheit 9 weitergeleitet, welche dazu ausgebildet ist, die Messdaten auszuwerten. So wertet die Auswerteeinheit 9 aus den Messdaten die Art und den Ort des Greifens mittels der Hände der Bedienperson aus. Sie kann zum Beispiel auswerten, ob die Bedienperson mit einer oder beiden Händen greift, wie viele Finger beteiligt sind, wie fest der Griff ist, wo genau gegriffen wird und wie stark in welche Richtung gedrückt wird und/oder kann z.B. ein Griffprofil erstellen.

Alternativ kann das Griffelement 7 für eine noch genauere Charakterisierung der Art des Greifens bzw. eines Griffprofils der Bedienperson weitere Sensoren aufweisen, z.B. Drucksensoren 15, resistive Sensoren, Näherungssensoren, Lichtsensoren oder Kamerasensoren 16. Auch die Messdaten dieser Sensoren werden von der Auswerteeinheit 9 hinsichtlich der Art und des Ortes des Greifens ausgewertet. Außerdem kann auch noch die Bedienperson selbst erkannt werden, z.B. anhand ihrer Handlungsmuster oder auch mittels Gesichtserkennung oder Fingerabdruckerkennung. Anschließend an die Auswertung der Messdaten hinsichtlich der Art und/oder des Ortes des Greifens durch die Bedienperson ordnet die Auswerteeinheit 9 die ermittelten Charakteristiken einem der vorhandenen Bewegungsmodi zu. Dieser identifizierte Bewegungsmodus wird dann als der durch die Bedienperson intendierte Bewegungsmodus interpretiert, an die Systemsteuerung 8 des mobilen C-Bogen-Röntgengeräts 1 weitergeleitet und mittels der Systemsteuerung 8 eingestellt. Auf diese Weise kann der Gerätewagen dann in dem eingestellten Bewegungsmodus bewegt werden. Damit lassen sich typische kontextbezogene Handlungsmuster der Bedienperson in passende Bewegungen des Gerätes umsetzen, ohne dass der Nutzer die intendierte Bewegung manuell (z.B. per Knopf oder Hebel) auswählen muss. Zusätzlich sind noch personalisierte Bewegungsprofile bei Erkennung der Bedienperson möglich.

Um der Bedienperson anzuzeigen, welcher Bewegungsmodus eingestellt ist bzw. in welchem Status sich die Handerkennung befindet, sind Anzeigeelemente 13 vorgesehen, z.B. LEDs oder kleine Bildschirme. Diese können im Umfeld des Griffelements 7 oder an einer anderen Stelle des mobilen C-Bogen-Röntgengeräts 1 angeordnet sein. Zusätzlich kann auch eine Anzeige an einem üblichen Monitor vorgesehen sein. Der Monitor 11 kann an dem mobilen C-Bogen-Röntgengerät oder an einem zusätzlichen mobilen Trolley 10 angeordnet sein.

Statt eines für das Greifen mit zwei Händen nutzbaren Griffelements 7 können z.B. auch zwei kleinere Griffelemente mit den entsprechenden Sensoren vorhanden sein, welche jeweils nur für das Greifen mit einer Hand vorgesehen sind. Die entsprechenden Messdaten können dann für beide Griffelemente gemeinsam ausgewertet werden, also auch z.B. ob nur mit einer Hand gegriffen wird und welche Art des Greifens vorliegt.

Neben der Verfahrbarkeit kann auch optional die Verstellbarkeit des C-Bogens 3 auf die beschriebene Weise automatisch eingestellt werden. So können auch hier zwei oder mehr Verstellmodi für die Verstellung des C-Bogens vorhanden sein, also bestimmte Voreinstellungen, bei denen jeweils ein oder mehrere Charakteristiken der Verstellung des C-Bogens (z.B. langsam, schnell, Rotation ... ) besonders unterstützt und/oder andere weniger oder gar nicht unterstützt bzw. blockiert werden. Der C-Bogen selbst ist servounterstützt manuell verstellbar. An einem weiteren Griffelement 17, welches für die manuelle, servounterstützte Verstellung des C-Bogens durch eine Bedienperson vorgesehen ist, sind ebenfalls kapazitive Sensoren 12 und/oder weitere Sensoren angeordnet, welche Messwerte erfassen, welche die Art und/oder den Ort des Greifens der Bedienperson charakterisieren. Die Messwerte können anschließend durch die Auswertungseinheit hinsichtlich der Art und/oder des Ortes des Greifens ausgewertet und einem Verstellmodus des C-Bogens zugeordnet werden. Der Verstellmodus wird anschließend automatisch durch die Systemsteuerung eingestellt.

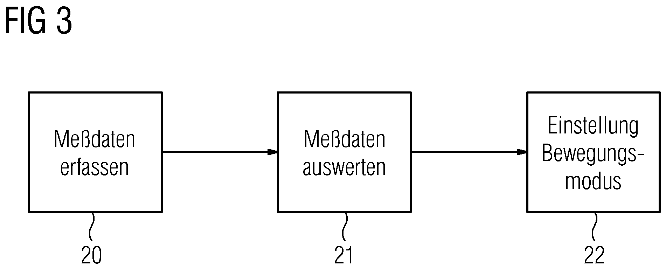

In der FIG 3 ist das erfindungsgemäße Verfahren zur Bedienunterstützung des mobilen C-Bogen-Röntgengeräts aus FIG 1 gezeigt. In einem ersten Schritt 20 werden von den Sensoren (12, 15, 16) Messdaten erfasst, welche die Art und/oder den Ort des Greifens mittels der mindestens einen menschlichen Hand charakterisieren. Anschließend werden in einem zweiten Schritt 21 die Messdaten hinsichtlich der Art und/oder des Ortes des Greifens ausgewertet und einem Bewegungsmodus des Gerätewagens zugeordnet. In einem dritten Schritt 22 wird der ermittelte Bewegungsmodus automatisch eingestellt.

Durch die Erfindung ist eine effizientere und intuitivere Bedienung der Plattform möglich, da nun eine Blindbedienung durch die Bedienperson gewährleistet ist. Der Bedienperson ist stets klar, welcher Bewegungsmodus eingestellt ist, da sie das direkt durch ihre Art des Greifens steuert. Das Verfahren zur automatischen Bedienunterstützung ist idealerweise in die Steuerungssoftware des mobilen C-Bogen-Röntgengeräts implementiert.

Alternativ können die verschiedenen Bewegungsmodi manuell z.B. über Schalter oder über ein User Interface eingestellt werden.

Außerdem kann ein mobiles Gerät mit einem in mindestens zwei Bewegungsmodi motorisch unterstützt verfahrbaren Gerätewagen aufweisend mindestens ein Griffelement, wobei das mindestens eine Griffelement für ein Greifen mittels mindestens einer menschlichen Hand einer Bedienperson zum Verfahren des Gerätes vorgesehen ist, und außerdem aufweisend mindestens einen Sensor, welcher an dem Griffelement oder in dessen unmittelbarer Nähe angeordnet und dafür ausgebildet ist, die Art und/oder den Ort des Greifens mittels der mindestens einen menschlichen Hand charakterisierende Messdaten zu erfassen, eine Auswerteeinheit, welche dazu ausgebildet ist, die Messdaten hinsichtlich der Art und/oder des Ortes des Greifens auszuwerten und einem Bewegungsmodus des Gerätewagens zuzuordnen, und eine Steuerungseinheit zur automatischen Einstellung des ausgewerteten Bewegungsmodus des Gerätewagens, auch eine nicht-medizinische Anwendung aufweisen. Bei dem mobilen Gerät kann es sich zum Beispiel um einen Einkaufswagen oder eine Lager-Transport-Plattform handeln. Insbesondere ist die Erfindung anwendbar für mobile Geräte mit hohem spezifischem Gewicht, um eine einfache Manövrierbarkeit zu gewährleisten.

So kann z.B. bei einem Einkaufswagen ein erster Bewegungsmodus bei Einhandbedienung (normales Verfahren) und ein zweiter Bewegungsmodus bei Zweihandbedienung (gebremstes Verfahren) eingestellt werden. Durch die Unterscheidung zwischen der Ein- und Zweihandbedienung kann z.B. auf abschüssigem Gelände (z.B. Parkplatz) ein sicheres Bewegen sichergestellt werden. Auf diese Weise kann der Einkaufswagen unabhängig vom Gewicht präzise und einfach gesteuert werden. Im Fall einer Lager-Transport-Plattform können z.B. sehr hohe Gewichte präzise bewegt werden, da in Logistikzentren viele Transportfahrzeuge noch manuell bewegt werden müssen. So kann hier zwischen einem ersten Bewegungsmodus (normales Verfahren auf dem Boden) bei Zweihandbedienung und einem zweiten Bewegungsmodus (Verfahren parallel an einem Regal entlang) bei Einhandbedienung unterschieden werden.

Die Erfindung lässt sich in folgender Weise kurz zusammenfassen: Mobiles medizinisches Gerät, insbesondere mobiles C-Bogen-Röntgengerät, mit einem in mindestens zwei Bewegungsmodi motorisch unterstützt verfahrbaren Gerätewagen, aufweisend mindestens ein Griffelement, wobei das mindestens eine Griffelement für ein Greifen mittels mindestens einer menschlichen Hand einer Bedienperson zum Verfahren des Gerätes vorgesehen ist, und außerdem aufweisend mindestens einen Sensor, welcher an dem Griffelement oder in dessen unmittelbarer Nähe angeordnet und dafür ausgebildet ist, die Art und/oder den Ort des Greifens mittels der mindestens einen menschlichen Hand charakterisierende Messdaten zu erfassen, eine Auswerteeinheit, welche dazu ausgebildet ist, die Messdaten hinsichtlich der Art und/oder des Ortes des Greifens auszuwerten und einem Bewegungsmodus des Gerätewagens zuzuordnen, und eine Steuerungseinheit zur automatischen Einstellung des ausgewerteten Bewegungsmodus des Gerätewagens. Die mindestens zwei Bewegungsmodi sind für mindestens zwei Bedienpersonen personalisiert einstellbar, wobei der mindestens eine Sensor dazu ausgebildet ist, ein Greifprofil einer Bedienperson charakterisierende Messdaten zu erfassen, die Auswerteeinheit dazu ausgebildet ist, die Messdaten hinsichtlich der Bedienperson auszuwerten und einem personalisierten Bewegungsmodus zuzuordnen und die Steuerungseinheit zur automatischen Einstellung des ausgewerteten personalisierten Bewegungsmodus ausgebildet ist.

## Patentansprüche

1. Mobiles medizinisches Gerät (1) mit einem in mindestens zwei Bewegungsmodi motorisch unterstützt verfahrbaren Gerätewagen (2), aufweisend mindestens ein Griffelement (7), wobei das mindestens eine Griffelement (7) für ein Greifen mittels mindestens einer menschlichen Hand einer Bedienperson zum Verfahren des Gerätes vorgesehen ist, und außerdem aufweisend
• mindestens einen Sensor (12, 15, 16), welcher an dem Griffelement (7) oder in dessen unmittelbarer Nähe angeordnet und dafür ausgebildet ist, die Art und/oder den Ort des Greifens mittels der mindestens einen menschlichen Hand charakterisierende Messdaten zu erfassen,
• eine Auswerteeinheit (9), welche dazu ausgebildet ist, die Messdaten hinsichtlich der Art und/oder des Ortes des Greifens auszuwerten und einem Bewegungsmodus des Gerätewagens (2) zuzuordnen, und
• eine Steuerungseinheit zur automatischen Einstellung des ausgewerteten Bewegungsmodus des Gerätewagens (2),
**dadurch gekennzeichnet, dass**
die mindestens zwei Bewegungsmodi für mindestens zwei Bedienpersonen personalisiert einstellbar sind, wobei der mindestens eine Sensor (12, 15, 16) dazu ausgebildet ist, ein Greifprofil einer Bedienperson charakterisierende Messdaten zu erfassen, die Auswerteeinheit (9) dazu ausgebildet ist, die Messdaten hinsichtlich der Bedienperson auszuwerten und einem personalisierten Bewegungsmodus zuzuordnen und die Steuerungseinheit zur automatischen Einstellung des ausgewerteten personalisierten Bewegungsmodus ausgebildet ist.

2. Mobiles medizinisches Gerät nach Anspruch 1, wobei der Sensor oder die Sensoren (12, 15, 16) dazu ausgebildet sind, die Anzahl der greifenden Hände und/oder die Größe der greifenden Hand oder Hände und/oder die Position der greifenden Hand oder Hände und/oder die Auflagefläche der greifenden Hand oder Hände und/oder die Grifffestigkeit des Greifens und/oder ein Greifprofil einer Bedienperson charakterisierende Messdaten zu erfassen.

3. Mobiles medizinisches Gerät Anspruch 1 oder 2, wobei der zumindest eine Sensor von zumindest einem kapazitiven Sensor (12) gebildet wird.

4. Mobiles medizinisches Gerät nach einem der vorangehenden Ansprüche, welches von einem mobilen C-Bogen-Röntgengerät (1) mit einem in mehreren Freiheitsgraden verstellbaren C-Bogen (3) gebildet wird.

5. Mobiles medizinisches Gerät nach Anspruch 4, wobei ein erstes Griffelement (7) zum Anschieben und/oder Ziehen des in mindestens zwei Bewegungsmodi motorisch unterstützt verfahrbaren Gerätewagens und ein zweites Griffelement (17) für ein Greifen mittels mindestens einer menschlichen Hand einer Bedienperson zum motorisch unterstützten Verstellen des C-Bogens (3) ausgebildet ist.

6. Mobiles medizinisches Gerät nach Anspruch 5, wobei das zweite Griffelement (17) mindestens einen Sensor (12) aufweist, welcher an dem Griffelement (17) oder in dessen unmittelbarer Nähe angeordnet und dafür ausgebildet ist, die Art und/oder den Ort des Greifens mittels der mindestens einen menschlichen Hand charakterisierende Messdaten zu erfassen, und wobei die Auswerteeinheit (9) dazu ausgebildet ist, die Messdaten hinsichtlich der Art und/oder des Ortes des Greifens auszuwerten und einem Verstellmodus des C-Bogens zuzuordnen, und die Steuerungseinheit zur automatischen Einstellung des ausgewerteten Verstellmodus ausgebildet ist.

7. Mobiles medizinisches Gerät nach einem der vorangehenden Ansprüche, aufweisend eine Vielzahl von Sensoren (12, 15, 16), welche an dem mindestens einen Griffelement oder in dessen unmittelbarer Nähe angeordnet sind.

8. Mobiles medizinisches Gerät nach einem der vorangehenden Ansprüche, wobei ein erster Bewegungsmodus derart ausgebildet ist, dass bei Einstellen des ersten Bewegungsmodus ein schnelles Verfahren des Gerätewagens (2) über Entfernungen > 4 m unterstützt wird.

9. Mobiles medizinisches Gerät nach einem der vorangehenden Ansprüche, wobei ein zweiter Bewegungsmodus derart ausgebildet ist, dass bei Einstellen des zweiten Bewegungsmodus ein langsames Verfahren des Gerätewagens (2) über Entfernungen < 50 cm unterstützt wird.

10. Mobiles medizinisches Gerät nach einem der vorangehenden Ansprüche, aufweisend eine Anzeigeelement (13) zur Anzeige von Informationen bezüglich des automatisch aktivierten Bewegungsmodus.

11. Verfahren zur automatischen Bedienunterstützung eines mobilen medizinischen Gerätes mit einem in mindestens zwei Bewegungsmodi motorisch unterstützt verfahrbaren Gerätewagen (2) und zumindest einem Griffelement (7) für ein Greifen mittels der mindestens einen menschlichen Hand einer Bedienperson zum Verfahren des Gerätewagens (2) mit den folgenden Schritten:
• Erfassen von die Art und/oder den Ort des Greifens mittels der mindestens einen menschlichen Hand charakterisierenden Messdaten,
• Auswerten der Messdaten hinsichtlich der Art und/oder des Ortes des Greifens und Zuordnen zu einem Bewegungsmodus des Gerätewagens (2), und
• Automatische Einstellung des ermittelten Bewegungsmodus des Gerätewagens (2),
• wobei die mindestens zwei Bewegungsmodi für mindestens zwei Bedienpersonen personalisiert einstellbar sind, wobei ein Greifprofil einer Bedienperson charakterisierende Messdaten erfasst werden, die Messdaten hinsichtlich der Bedienperson ausgewertet und einem personalisierten Bewegungsmodus zugeordnet werden und der ausgewertete personalisierte Bewegungsmodus automatisch eingestellt wird.

12. Verfahren nach Anspruch 11, wobei die Anzahl der greifenden Hände und/oder die Größe der greifenden Hand oder Hände und/oder die Position der greifenden Hand oder Hände und/oder die Auflagefläche der greifenden Hand oder Hände und/oder die Grifffestigkeit des Greifens und/oder ein Greifprofil einer Bedienperson charakterisierende Messdaten erfasst werden.

## Claims

1. Mobile medical device (1) having a device trolley (2) which can be moved in at least two movement modes in a motor-assisted manner, having at least one gripping element (7), wherein the at least one gripping element (7) is provided for a gripping by means of at least one human hand of an operating person for moving the device, and also having:
• at least one sensor (12, 15, 16) which is arranged on the gripping element (7) or in the direct vicinity thereof and is configured to capture measurement data characterising the manner and/or the location of the gripping by means of the at least one human hand,
• an evaluating unit (9) which is configured to evaluate the measurement data with regard to the manner and/or the location of the gripping and to associate it with a movement mode of the device trolley (2), and
• a control unit for automatic setting of the evaluated movement mode of the device trolley (2),
**characterised in that**
the at least two movement modes can be set in a personalised manner for at least two operating persons, wherein the at least one sensor (12, 15, 16) is configured to capture measurement data characterising a gripping profile of an operating person, the evaluating unit (9) is configured to evaluate the measurement data with regard to the operating person and to associate it with a personalised movement mode, and the control unit is configured for automatic setting of the evaluated personalised movement mode.

2. Mobile medical device according to claim 1, wherein the sensor or sensors (12, 15, 16) are configured to capture measurement data characterising the number of gripping hands and/or the size of the gripping hand or hands and/or the position of the gripping hand or hands and/or the contact area of the gripping hand or hands and/or the grip tightness of the gripping and/or a gripping profile of an operating person.

3. Mobile medical device according to claim 1 or 2, wherein the at least one sensor is formed by at least one capacitive sensor (12).

4. Mobile medical device according to one of the preceding claims which is formed by a mobile C-arm X-ray device (1) having a rotatable C-arm (3) which is adjustable in a plurality of degrees of freedom.

5. Mobile medical device according to claim 4, wherein a first gripping element (7) is configured for pushing and/or pulling the device trolley which can be moved in at least two movement modes in a motor-assisted manner and a second gripping element (17) for a gripping by means of at least one human hand of an operating person for motor-assisted adjustment of the C-arm (3).

6. Mobile medical device according to claim 5, wherein the second gripping element (17) has at least one sensor (12) which is arranged on the gripping element (17) or in the direct vicinity thereof and is configured to capture measurement data characterising the manner and/or the location of the gripping by means of the at least one human hand, and wherein the evaluating unit (9) is configured to evaluate the measurement data with regard to the manner and/or the location of the gripping and to associate it with an adjustment mode of the C-arm, and the control unit is configured for automatic setting of the evaluated adjustment mode.

7. Mobile medical device according to one of the preceding claims, having a plurality of sensors (12, 15, 16) which are arranged on the at least one gripping element or in the direct vicinity thereof.

8. Mobile medical device according to one of the preceding claims, wherein a first movement mode is configured such that on setting of the first movement mode, a rapid movement of the device trolley (2) over distances > 4 m is supported.

9. Mobile medical device according to one of the preceding claims, wherein a second movement mode is configured such that on setting the second movement mode, a slow movement of the device trolley (2) over distances < 50 cm is supported.

10. Mobile medical device according to one of the preceding claims, having a display element (13) for displaying information relating to the automatically activated movement mode.

11. Method for automatic operating assistance for a mobile medical device having a device trolley (2) which can be moved in at least two movement modes in a motor-assisted manner and at least one gripping element (7) for a gripping by means of at least one human hand of an operating person for moving the device trolley (2), comprising the following steps:
• capturing measurement data characterising the manner and/or the location of the gripping by means of the at least one human hand,
• evaluating the measurement data with regard to the manner and/or the location of the gripping and associating it with a movement mode of the device trolley (2), and
• automatic setting of the determined movement mode of the device trolley (2),
• wherein the at least two movement modes can be set in a personalised manner for at least two operating persons, wherein measurement data characterising a gripping profile of an operating person is captured, the measurement data is evaluated with regard to the operating person and is associated with a personalised movement mode, and the evaluated personalised movement mode is set automatically.

12. Method according to claim 11, wherein measurement data characterising the number of gripping hands and/or the size of the gripping hand or hands and/or the position of the gripping hand or hands and/or the contact area of the gripping hand or hands and/or the grip tightness of the gripping and/or a gripping profile of an operating person is captured.

## Revendications

1. Appareil (1) médical mobile comprenant un chariot (2) d'appareil pouvant être déplacé avec soutien motorisé dans au moins deux modes de déplacement, comportant au moins une poignée (7), dans lequel la au moins une poignée (7) est prévue pour une préhension au moyen d'au moins la main humaine d'une personne de service, pour le déplacement de l'appareil, et comportant en outre
• au moins un capteur (12, 15, 16), qui est monté sur la poignée (7) ou dans sa proximité immédiate et qui est constitué pour détecter des données de mesure caractérisant le type et/ou l'emplacement de la préhension au moyen de la au moins une main humaine,
• une unité (9) d'évaluation, qui est constituée pour évaluer les données de mesure en ce qui concerne le type et/ou l'emplacement de la préhension et pour les affecter à un mode de déplacement du chariot (2) d'appareil, et
• une unité de commande pour l'établissement automatique du mode de déplacement évalué du chariot (2) d'appareil,
**caractérisé en ce que**
les au moins deux modes de déplacement sont réglables d'une manière personnalisée pour au moins deux personnes de service, dans lequel le au moins un capteur (12, 15, 16) est constitué pour détecter des données de mesure caractérisant un profil de préhension d'une personne de service, l'unité (9) d'évaluation est constituée pour évaluer les données de mesure en ce qui concerne la personne de service et pour les associer à un mode de déplacement personnalisé et l'unité de commande est constituée pour l'établissement automatique du mode de déplacement personnalisé évalué.

2. Appareil médical mobile suivant la revendication 1, dans lequel le capteur ou les capteurs (12, 15, 16) sont constitués pour détecter des données de mesure caractérisant le nombre des mains prenantes et/ou la dimension de la main ou des mains prenantes et/ou la position de la main ou des mains prenantes et/ou la surface d'application de la main ou des mains prenantes et/ou la solidité de la préhension et/ou un profil de préhension d'une personne de service.

3. Appareil médical mobile suivant la revendication 1 ou 2, dans lequel le au moins un capteur est formé d'au moins un capteur (12) capacitif.

4. Appareil médical mobile suivant l'une des revendications précédentes, qui est formé d'un appareil (1) de rayons X à arceau en C mobile ayant un arceau en C (3) pouvant être déplacé suivant plusieurs degrés de liberté.

5. Appareil médical mobile suivant la revendication 4, dans lequel un premier élément (7) de poignée est constitué pour pousser et/ou tirer le au moins un chariot d'appareil pouvant être déplacé avec soutien motorisé dans au moins deux modes déplacement et un deuxième élément (17) de poignée est constitué, pour le déplacement avec soutien motorisé de l'arceau en C (3), pour une préhension au moyen d'au moins la main humaine d'une personne de service.

6. Appareil médical mobile suivant la revendication 5, dans lequel le deuxième élément (17) de poignée a au moins un capteur (12), qui est monté sur l'élément (17) de poignée ou dans sa proximité immédiate et qui est constitué pour détecter des données de mesure caractérisant le type et/ou l'emplacement de la préhension au moyen de la au moins une main humaine, et dans lequel l'unité (9) d'évaluation est constituée pour évaluer les données de mesure en ce qui concerne le type et/ou l'emplacement de la préhension et les affecter à un mode de déplacement de l'arceau en C, et l'unité de commande est constituée pour l'établissement automatique du mode de déplacement évalué.

7. Appareil médical mobile suivant l'une des revendications précédentes, comportant une pluralité de capteurs (12, 15, 16), qui sont montés sur la au moins un élément de préhension ou dans sa proximité immédiate.

8. Appareil médical mobile suivant l'une des revendications précédentes, dans lequel un premier mode de déplacement est constitué, de façon à venir à l'appui, lors de l'établissement du premier mode de déplacement, d'un déplacement rapide du chariot (2) d'appareil à des éloignements > 4 m.

9. Appareil médical mobile suivant l'une des revendications précédentes, dans lequel un deuxième mode de déplacement est constitué, de manière à venir à l'appui, lors de l'établissement du deuxième mode de déplacement, d'un déplacement lent du chariot (2) d'appareil à des éloignements < 50 cm.

10. Appareil médical suivant l'une des revendications précédentes, comportant un élément (13) d'affichage pour l'affichage d'informations concernant le mode de déplacement activé automatiquement.

11. Procédé pour venir à l'appui du fonctionnement automatique d'un appareil médical mobile ayant un chariot (2) d'appareil déplaçable avec soutien motorisé dans au moins deux modes de déplacement et au moins un élément (7) de poignée pour une préhension au moyen d'au moins la main humaine d'une personne de service pour le déplacement du chariot (2) d'appareil, comprenant les stades suivants :
• détection du type et/ou de l'emplacement de la préhension au moyen de données de mesure caractérisant au moins une main humaine,
• évaluation des données de mesure en ce qui concerne le type et/ou l'emplacement de la préhension et leur affectation à un mode de déplacement du chariot (2) d'appareil, et
• établissement automatique du mode de déplacement déterminé du chariot (2) d'appareil,
• dans lequel les au moins deux modes de déplacement sont réglables de manière personnalisée pour au moins deux personnes de service, dans lequel on détecte des données de mesure caractérisant un profil de préhension d'une personne de service, on évalue les données de mesure en ce qui concerne la personne de service et on les associe à un mode de déplacement personnalisé et on établit automatiquement le mode de déplacement personnalisé évalué.

12. Procédé suivant la revendication 11, dans lequel on détecte des données de mesure caractérisant le nombre des mains prenantes et/ou la dimension de la main ou des mains prenantes et/ou la position de la main ou des mains prenantes et/ou la surface d'application de la main ou des mains prenantes et/ou la solidité de la préhension et/ou un profil de préhension d'une personne de service.
